(19) Europäisches Patentamt
European Patent Office
Office européen des brevets

(11) Publication number: **0 317 728 A2**

(12) **EUROPEAN PATENT APPLICATION**

(21) Application number: **88115479.3**

(51) Int. Cl.4: **A61K 31/70**

(22) Date of filing: **21.09.88**

(30) Priority: **22.09.87 US 99562**

(43) Date of publication of application:
**31.05.89 Bulletin 89/22**

(84) Designated Contracting States:
**AT BE CH DE ES FR GB GR IT LI LU NL SE**

(71) Applicant: **WARNER-LAMBERT COMPANY**
**201 Tabor Road**
**Morris Plains New Jersey 07950(US)**

(72) Inventor: **Cook, Phillip Dan**
**3 Surrey Lane**
**Downingtown Pennsylvania 19335(US)**

(74) Representative: **Mansmann, Ivo**
**c/o Gödecke AG - Patentabteilung Postfach**
**569 - Mooswaldallee 1-9**
**D-7800 Freiburg(DE)**

(54) **Use of 9-(beta-D-arabinofuranosyl) adenine derivatives for the preparation of pharmaceutical compositions for the treatment of aids.**

(57) 9-(β-D-arabinofuranosyl)adenine, also commonly known as Ara-A or vidarabine, esters thereof, or a pharmaceutically acceptable salt thereof, possess antiviral activity against the HTLV-III/LAV virus implicated in acquired immune deficiency syndrome (AIDS). The invention is related to the use of compounds of formula I

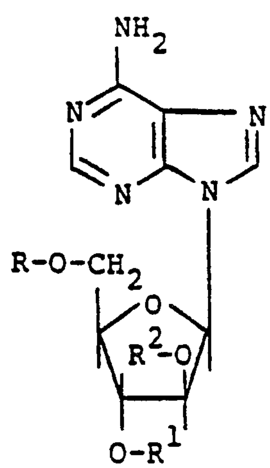

I

for the preparation of pharmaceutical compositions useful in the treatment of infections of the HTLV-III/LAV virus in patients.

EP 0 317 728 A2

# USE OF 9-(β-D-ARABINOFURANOSYL) ADENINE DERIVATIVES FOR THE PREPARATION OF PHARMACEUTICAL COMPOSITIONS FOR THE TREATMENT OF AIDS

The present invention concerns the use of 9-(β-D-arabinofuranosyl)adenine, esters thereof, or a pharmaceutically acceptable salt thereof for the preparation of pharmaceutical compositions for the treatment of infections of the HTLV-III/LAV virus in patients.

9-(β-D-arabinofuranosyl)adenine also commonly known as Ara-A or vidarabine, is described in United States Patent 3,616,208. The compound is disclosed there as having in vitro and in vivo activity against both herpes and vaccinia viruses.

The following esters and salts thereof of 9-(β-D-arabinofuranosyl)adenine also have been disclosed as having in vitro activity against herpes simplex virus:

9-(β-D-arabinofuranosyl)adenine, 5′ phosphate, also commonly known as Ara-AMP, and salts thereof are described in United States patents 3,703,507 and 4,123,609;

9-(3-O-acyl-β-D-arabinofuranosyl)adenine and 9-(2,3-di-O-acyl-β-D-arabinofuranosyl)adenine compounds are described in United States patent 4,055,717;

9-(2-O-acyl-β-D-arabinofuranosyl)adenine compounds are described in United States patents 4,055,718 and 4,212,941;

9-(3,5-di-O-acyl-β-D-arabinofuranosyl)adenine compounds are described in United States patent 4,048,432;

9-(5-O-acyl-β-D-arabinofuranosyl)adenine compounds are described in United States patent 4,069,382;

9-(β-D-arabinofuranosyl)adenine, triester compounds are described in United States patent 3,651,045.

Acquired immune deficiency syndrome (AIDS) is a progressive degenerative disease of both the immune system and the central nervous system. The death of AIDS patients frequently results due to opportunistic infections which overwhelm the body once the immune system has been compromised.

Human T-cell lymphotropic virus type III, lymphadenopathy-associated virus (HTLV-III/LAV) is currently accepted as the infectious agent that causes AIDS, although other, still-unidentified cofactors may also be necessary for the expression of the disease.

The disease is worldwide and spreading rapidly. In May of 1987 there were 49,677 reported cases of AIDS worldwide in 112 countries, representing an increase of 6.5% over the number of cases reported one month earlier. It is estimated that the actual number of AIDS cases worldwide is as many as 100,000.

There is an urgent need for methods to combat this growing threat to health. A number of agents have been suggested for use in treating AIDS including 3′-azido-3′deoxythymidine (AZT), α-interferon, ampligen, and amphotericin B methyl ester.

Accordingly the present invention provides the use of a compound having the Formula I

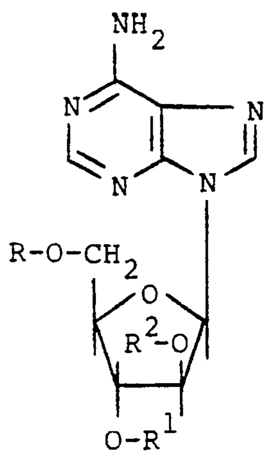

I

or a pharmaceutically acceptable acid addition salt thereof where

R is hydrogen, an alkanoyl group of from two to eight carbon atoms, or

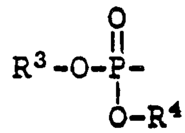

wherein $R^3$ and $R^4$ are each independently hydrogen, an alkali metal cation, an alkaline earth-metal cation or an ammonium cation;

$R^1$ and $R^2$ are each independently hydrogen, or an alkanoyl group of from two to eight carbon atoms for the preparation of a pharmaceutical composition for the treatment of infections of the HTLV-III/LAV virus in patients.

In the compounds of Formula I, the term "alkanoyl" is a

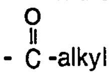 -alkyl group where "alkyl" is meant to include a straight or branched alkyl group having from one to eight carbon atoms such as, for example, methyl, ethyl, propyl, isopropyl, butyl,isobutyl, pentyl, 3-methylbutyl, 2,2-dimethylpropyl, hexyl, octyl, and the like.

The term "alkali metal" refers to a metal in Group 1A of the periodic table, such as, for example, lithium, sodium, potassium, and the like.

The term "alkaline-earth metal" refers to a metal in Group IIA of the periodic table, such as, for example, calcium, barium, and the like.

The compounds of Formula I are useful for the treatment of HTLV III/LAV infections both in the free base form and in the form of acid addition salts. The two forms are within the scope of the present invention. Pharmaceutically acceptable acid addition salts are formed with inorganic and organic acids, such as, for example, hydrochloric, sulfuric, phosphoric, acetic, citric, gluconic, fumaric, methanesulfonic and the like (see, for example, Berge, S. M., et al, "Pharmaceutical Salts", Journal of Pharmaceutical Science, 66, pp. 1-19 (1977)). The acid addition salts of said basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Further, the compounds of Formula I wherein R is a

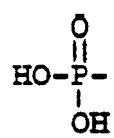

group are useful both in the free acid form and in the form of base addition salts. The two forms are within the scope of the present invention. Pharmaceutically acceptable base addition salts are formed with inorganic and organic bases, such as, for example, ammonium hydroxide, sodium hydroxide, lithium hydroxide and the like (see, for example, Berge, S. M., et al, "Pharmaceutical Salts", Journal of Pharmaceutical Science, 66, pp. 1-19 (1977)). The base addition salts of said acidic compounds are prepared by contacting the free acid form with a sufficient amount of the desired base to produce the salt in the conventional manner. The free acid form may be regenerated by contacting the salt form with an acid and isolating the free acid in the conventional manner. The free acid forms differ from their respective salt forms somewhat in certain physical properties such as solubility in polar solvents, but otherwise the salts are equivalent to their respective free acid for purposes of the present invention.

Certain of the compounds of the present invention can exist in unsolvated as well as solvated forms, including hydrated forms. In general, the solvated forms, including hydrated forms are equivalent to unsolvated forms and are intended to be encompassed within the scope of the present invention.

Illustrative examples of compounds falling within the scope of the present invention are the following:

9-(β-D-arabinofuranosyl)adenine;

9-(β-D-arabinofuranosyl)adenine, 5′-phosphate;

9-(3-O-acetyl-β-D-arabinofuranosyl)adenine;

9-(3-O-propionyl-β-D-arabinofuranosyl)adenine;

9-(3-O-isobutyryl-β-D-arabinofuranosyl)adenine;

9-(2,3-di-O-acetyl-β-D-arabinofuranosyl)adenine;

9-(2,3-di-O-propionyl-β-D-arabinofuranosyl)-adenine;
9-(2,3-di-O-isobutyryl-β-D-arabinofuranosyl)-adenine;
9-(2-O-acetyl-(β-D-arabinofuranosyl)adenine;
9-(2-O-propionyl-β-D-arabinofuranosyl)adenine;
9-(2-O-isobutyryl-β-D-arabinofuranosyl)adenine;
9-(3,5-di-O-propionyl-β-D-arabinofuranosyl)-adenine; 9-(3,5-di-O-isobutyryl-β-D-arabinofuranosyl-adenine; 9-(3,5-di-O-acetyl-β-D-arabinofuranosyl)adenine;
9-(3,5-di-O-butyryl-β-D-arabinofuranosyl)adenine;
9-(5-0-acetyl-β-D-arabinofuranosyl)adenine;
9-(5-O-propionyl-β-D-arabinofuranosyl)adenine;
9-(5-O-butyryl-β-D-arabinofuranosyl)adenine;
9-(5-O-pentanoyl-β-D-arabinofuranosyl)adenine;
9-(5-O-3-methylbutyryl-β-D-arabinofuranosyl)adenine;
9-(5-O-2,2-dimethylpropionyl-β-D-arabinofuranosyl)adenine;
9-(5-O-hexanoyl-β-D-arabinofuranosyl)adenine;
9-(5-O-octanoyl-β-D-arabinofuranosyl)adenine;
9-(β-D-arabinofuranosyl)adenine, triacetate ester;
and
9-(β-D-arabinofuranosyl)adenine, tripropionate ester; or a pharmaceutically acceptable salt thereof.

The antiviral activity of the compounds of the present invention against the HTLV-III/LAV virus was evaluated by determining the degree of inhibition of cytopathic effect (CPE) and reverse transcriptase (RT) activity of the drug in a subline of H9 cells at various concentrations.

HTLV-III/LAV inoculum was standardized for reverse transcriptase (RT) activity using purified avian myeloblastosis viral RT (BRL Labs, Gaithersberg, MD) and used to infect polybrene treated H9 cells at 0.02 RT units of HTLV-III/LAV per 2 x $10^6$ cells. The virus was adsorbed for two hours at 37° C and then the cells were washed and resuspended in RPMI-10% fetal bovine serum at 2 x $10^5$ cells/ml and dispersed in 1-ml aliquots into 24-well plates.

Syncytial giant cell formation appeared at 5-6 days post infection, and this cytopathic effect (CPE) was quantitatively measured by dispersing 0.1-ml aliquots into 0.1-ml portions of absolute methanol and enumerating the giant cells microscopically. The inhibition of cytopathic effect by treatment with representative compounds of the present invention is shown in the table, together with the inhibitory effect on reverse transcriptase activity.

Reverse transcriptase activity (RT) was measured using 1-ml culture aliquots which were clarified at 600 xg for 30 minutes, precipitated in 10% polyethylene glycol-0.13 M NaCl at 4° C for eighteen hours, and centrifuged at 600 xg for sixty minutes. The pellet was dissolved in glycerol-Tris buffer (50% glycerol, 25 mM Tris HCl, pH 7.5, 5 mM dithiothreitol, 15 mM KCl, 0.025% Triton-X, and 0.25 mM EDTA). The RT assay was adapted from the methods of J. Levy, et al, Science, 225: 840 (1984), and of D. D. Ho, et al, Science, 226: 451 (1984) using a final reaction mixture containing 40 mM Tris-HCl, pH 7.8, 2.2 mM dithiothreitol, 10 mM $MgCl_2$, 50 mM KCl, 0.03% Triton-X, 25 μCi $^3$H-thymidine triphosphate (New England Nuclear, Boston, MA), and 50 μg/ml poly rA oligo $dT_{12-18}$ (BRL, Gaithersburg, MD). Background counts were determined using poly dA oligo $dT_{12-18}$ as template and subtracted from the poly rA dT primer to determine the thymidine incorporation specifically due to reverse transcriptase activity.

Table

| Effect of Compounds of Formula I on HLTV/III-LAV | | | |
|---|---|---|---|
| Compound | CPE* | RT** | Cell Number*** |
| | 50% Inhibitory Concentrations (μM) | | |
| 9-(β-D-arabinofuranosyl)adenine | 7 | 7 | >100 |
| 9-(β-D-arabinofuranosyl)adenine, 5′-phosphate | 18 | 11 | >100 |

* Concentration of compound at which the cytopathic effect (syncytial giant cell formation) on day 7 post infection was 50% that in drug-free control cultures.

** Concentration of compound at which the reverse transcriptase in the culture medium on day 10 post infection was 50% that in drug-free control cultures.

*** Concentration of compound at which the number of uninfected H9 cells present at day 6 was reduced to 50% of that in drug-free control cultures.

The compounds of the invention can be prepared and administered in a wide variety of oral and parenteral dosage forms. It will be obvious to those skilled in the art that the following dosage forms may comprise as the active component, either a compound of Formula I or a corresponding pharmaceutically acceptable salt of a compound of Formula I.

For preparing pharmaceutical compositions from the compounds of the present invention, pharmaceutically acceptable carriers can be either solid or liquid. Solid form preparations include powders, tablets, pills, capsules, dispersible granules, cachets, and suppositories. A solid carrier can be one or more substances which may also act as diluents, flavoring agents, solubilizers, lubricants, suspending agents, binders, preservatives, tablet disintegrating agents or an encapsulating material.

In powders, the carrier is a finely divided solid which is in a mixture with the finely divided active component.

In tablets, the active compound is mixed with the carrier having the necessary binding properties in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component, with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool and thereby to solidify.

Liquid form preparations include solutions, suspensions, and emulsions, for example, water or water propylene glycol solutions.

Aqueous solutions suitable for oral use can be prepared by dissolving the active component in water and adding suitable colorants, flavors, stabilizing, and thickening agents as desired.

Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form, the preparation is subdivided into unit doses containing appropriate quantities of the active component. The unit dosage form can be a packaged preparation, the package containing discrete quantities of preparation, such as packeted

tablets, capsules, and powders in vials or ampoules. Also, the unit dosage form can be a capsule, tablet, cachet or lozenge itself or it can be the appropriate number of any of these in packaged form.

A pharmaceutical composition prepared according to the present invention or one of its pharmaceutically acceptable salts may be generally administered to the patient in need of treatment in the form of an intravenous formulation. Certain of the ester derivatives of the present invention may be administered in oral dosage form.

Sterile solutions of compounds of the present invention suitable for injection are formed by dissolving the compound or desired salt form in a sterile solvent and subsequently sealing the sterile solution in vials or ampoules. Alternatively, the solution can be prepared and then subsequently sterilized by passage through micropore filters for sterilization.

Suitable solvents for the preparation of sterile parenteral solutions include pyrogen-free sterile solvents containing water, ethanol, polyols (e.g., glycerol, propylene glycol, liquid polyethylene glycol, and the like), N,N-dimethylacetamide, suitable mixtures thereof, and sterile vegetable oils.

Sterile powders for the reconstitution as injectable formulations are prepared by vacuum or freeze-drying previously prepared sterile solutions.

Parenteral preparations may contain from 0.1 to about 500 mg/ml of the active component, with 0.5 to about 250 mg/ml being preferred.

In use for treating infections of HTLV-III/LAV virus, a compound of the present invention is administered daily for five to ten days in doses ranging between about 10 mg/kg/day and 15 mg/kg/day, repeating the treatment every four weeks as needed. The particular dose employed is varied, however, depending upon a number of factors including the previous medical history and condition of the patient, the severity of the condition being treated, and the potency of the drug being administered. Determination of the appropriate dosage for a particular situation is within the skill of the art. Generally, treatment is started with lower doses, increasing the dose until the desired effect is achieved.

## Claims

1. Use of a compound having the Formula I

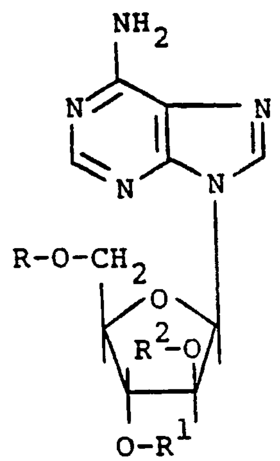

I

or a pharmaceutically acceptable acid addition salt thereof where R is hydrogen, an alkanoyl group of from two to eight carbon atoms, or

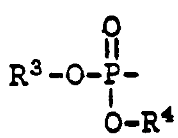

wherein

$R^3$ and $R^4$ are each independently hydrogen, an alkali metal cation, an alkaline-earth metal cation or an ammonium cation;

$R^1$ and $R^2$ are each independently hydrogen, or an alkanoyl group of from two to eight carbon atoms for the preparation of a pharmaceutical composition for the treatment of infections of the HTLV-III/LAV virus in patients.

2. Use according to Claim 1 wherein said compound of Formula I is 9-(β-D-arabinofuranosyl) adenine or 9-(β-D-arabinofuranosyl)adenine, 5′-phosphate.

3. Use according to Claim 1 wherein said compound of Formula I is 9-(2,3-di-O-acetyl-β-D-arabinofuranosyl)adenine.

4. Use according to Claim 1 wherein said compound of Formula I is 9-(5-O-pentanoyl-β-D-arabinofuranosyl)adenine.

5. Use according to Claim 1 wherein said compound of Formula I is used for preparing a parenteral composition.

6. Use according to Claim 1 wherein said compound of Formula I is used for preparing an intravenous composition.

7. Use according to Claim 2 wherein said 9-(β-D-arabinofuranosyl)adenine is used for preparing an intravenous composition.

8. Use according to Claim 2 wherein said 9-(β-D-arabinofuranosyl)adenine, 5′-phosphate is used for preparing an intravenous composition.

9. Process for the preparation of a pharmaceutical, which is useful for treating infections of the HTLV-III/LAV virus in patients, characterized by combining a compound having the Formula I

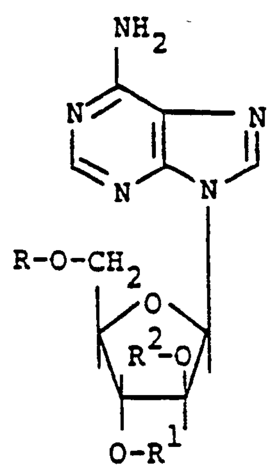

I

or a pharmaceutically acceptable acid addition salt thereof where R is hydrogen, an alkanoyl group of from two to eight carbon atoms, or

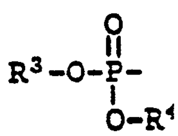

wherein

$R^3$ and $R^4$ are each independently hydrogen, an alkali metal cation, an alkaline-earth metal cation or an ammonium cation;

$R^1$ and $R^2$ are each independently hydrogen, or an alkanoyl group of from two to eight carbon atoms with a pharmaceutically acceptable carrier.

10. Process according to Claim 9, wherein the compound is a member of the group selected from the group consisting of

9-(β-D-arabinofuranosyl)adenine;

9-(β-D-arabinofuranosyl)adenine, 5′-phosphate;

9-(2,3-di-O-acetyl-β-D-arabinofuranosyl) adenine;

9-(5-O-pentanoyl-β-D-arabinofuranosyl)adenine.